# FASCICULE DE BREVET EUROPEEN

(11) **EP 1 532 102 B1**
(45) Date de publication et mention de la délivrance du brevet: **22.08.2018**
(21) Numéro de dépôt: 03761635.6
(22) Date de dépôt: 25.06.2003
(51) Int. Cl.: C07C 233/18, C07C 231/02, C07C 303/38, C07C 311/17, C07C 275/24, C07C 273/18, A61K 31/192, A61K 31/18, A61K 31/17, A61P 17/00

(54) **COMPOSITIONS COSMETIQUES OU DERMOPHARMACEUTIQUES COMPRENANT DES DERIVES DE LA TYRAMINE, LEUR PROCEDE DE PREPARATION, ET LEUR UTILISATION**
KOSMETISCHE ODER DERMOPHARMAZEUTISCHE ZUSAMMENSETZUNGEN ENTHALTEND TYRAMINDERIVATE, VERFAHREN ZUR DEREN HERSTELLUNG UND DEREN VERWENDUNG
COSMETIC OR DERMOPHARMACEUTICAL COMPOSITIONS COMPRISING TYRAMINE DERIVATIVES, METHOD FOR PREPARING SAME, AND USE THEREOF

(30) Priorité: 26.06.2002 FR 0207965
(43) Date de publication de la demande: 25.05.2005
(73) Titulaire: Sederma, 78612 Le Perray-en-Yvelines Cedex (FR)
(72) Inventeur: LINTNER, Karl, F-06500 Menton (FR)
(86) Numéro de dépôt international: PCT/FR2003/001950
(87) Numéro de publication internationale: WO 2004/002941

(56) Documents cités:
- FR-A- 2 813 188
- US-A- 4 258 058
- US-A- 4 515 773
- DATABASE CROSSFIRE BEILSTEIN [en ligne] Beilstein Institut zur Förderung der Chemischen Wissenschaften, Frankfurt am Main, DE; Database accession no. 3379913 XP002234001 & WALPOLE: JOURNAL OF THE CHEMICAL SOCIETY., vol. 97, 1910, page 946 CHEMICAL SOCIETY, LONDON, GB ISSN: 0368-1769
- DATABASE CROSSFIRE BEILSTEIN [en ligne] Beilstein Institut zur Förderung der Chemischen Wissenschaften, Frankfurt am Main, DE; Database accession no. 3410716 XP002234002 & RAO: JOURNAL OF THE INDIAN CHEMICAL SOCIETY., vol. 18, 1941, pages 316-319, THE INDIAN CHEMICAL SOCIETY, CALCUTTA., IN ISSN: 0019-4522
- DATABASE CROSSFIRE BEILSTEIN [en ligne] Beilstein Institut zur Förderung der Chemischen Wissenschaften, Frankfurt am Main, DE; Database accession no. 5880163 XP002234003 & ALIG, L ET AL: JOURNAL OF MEDICINAL CHEMISTRY., vol. 35, no. 23, 1992, pages 4393-4407, AMERICAN CHEMICAL SOCIETY., US ISSN: 0022-2623
- DATABASE CROSSFIRE BEILSTEIN [en ligne] Beilstein Institut zur Förderung der Chemischen Wissenschaften, Frankfurt am Main, DE; Database accession no. 8341940 XP002234004 & LIMA, L M ET AL: PHARMACEUTICA ACTA HELVETIAE., vol. 73, no. 6, 1999, pages 281-292, XX, XX ISSN: 0031-6865
- DATABASE CROSSFIRE BEILSTEIN [en ligne] Beilstein Institut zur Förderung der Chemischen Wissenschaften, Frankfurt am Main, DE; Database accession no. 8915882 XP002234005 & BARN, D R ET AL: BIOORGANIC & MEDICINAL CHEMISTRY., vol. 9, no. 10, 2001, pages 2609-2624, ELSEVIER SCIENCE LTD., GB ISSN: 0968-0896
- DATABASE CROSSFIRE BEILSTEIN [en ligne] Beilstein Institut zur Förderung der Chemischen Wissenschaften, Frankfurt am Main, DE; Database accession no. 6286015 XP002234006 & PARINI, C ET AL: STEROIDS, vol. 46, no. 4,5, - 1985 pages 903-914, BUTTERWORTH-HEINEMANN, STONEHAM, MA, US ISSN: 0039-128X
- DATABASE CROSSFIRE BEILSTEIN [en ligne] Beilstein Institut zur Förderung der Chemischen Wissenschaften, Frankfurt am Main, DE; Database accession no. 9058987 XP002234007 & SUN, WON-SUCK ET AL: JOURNAL OF ANTIBIOTICS., vol. 54, no. 10, 2001, pages 827-830, JAPAN ANTIBIOTICS RESEARCH ASSOCIATION. TOKYO., JP ISSN: 0021-8820
- DATABASE CROSSFIRE BEILSTEIN [en ligne] Beilstein Institut zur Förderung der Chemischen Wissenschaften, Frankfurt am Main, DE; Database accession no. 2774642 XP002234008 & TANAKA ET AL: YAKUGAKU ZASSHI - JOURNAL OF THE PHARMACEUTICAL SOCIETY OF JAPAN, vol. 87, 1967, pages 14-21, NIHON YAKUGAKKAI, TOKYO,, JP ISSN: 0031-6903
- DATABASE CROSSFIRE BEILSTEIN [en ligne] Beilstein Institut zur Förderung der Chemischen Wissenschaften, Frankfurt am Main, DE; Database accession no. 2805816 XP002234009 & TANAKA ET AL: YAKUGAKU ZASSHI - JOURNAL OF THE PHARMACEUTICAL SOCIETY OF JAPAN, vol. 87, 1967, pages 14-21, NIHON YAKUGAKKAI, TOKYO,, JP ISSN: 0031-6903
- DATABASE CROSSFIRE BEILSTEIN [en ligne] Beilstein Institut zur Förderung der Chemischen Wissenschaften, Frankfurt am Main, DE; Database accession no. 2882110 XP002234010 & RYCZEK, J: POLISH JOURNAL OF CHEMISTRY., vol. 70, no. 12, - 1996 pages 1518-1521, POLISH CHEMICAL SOCIETY., XX
- DATABASE CROSSFIRE BEILSTEIN [en ligne] Beilstein Institut zur Förderung der Chemischen Wissenschaften, Frankfurt am Main, DE; Database accession no. 8849001 XP002234011 & JORDAN, A M ET AL: BIOORGANIC & MEDICINAL CHEMISTRY., vol. 9, no. 6, 2001, pages 1549-1558, ELSEVIER SCIENCE LTD., GB ISSN: 0968-0896
- PATENT ABSTRACTS OF JAPAN vol. 2000, no. 16, 8 mai 2001 (2001-05-08) & JP 2001 019607 A (KANEBO LTD), 23 janvier 2001 (2001-01-23)

## Description

La présente divulgation concerne des compositions cosmétiques ou dermopharmaceutiques comprenant des dérivés de la tyramine, ainsi que leur procédé de préparation, et l'utilisation de ces compositions pour le soin de la peau, en particulier dans l'objectif de réduire l'hyperpigmentation.

La pigmentation naturelle de la peau relève d'un mécanisme aujourd'hui bien décrit : les mélanocytes présents dans la couche basale de l'épiderme produisent des pigments de mélanine, la synthèse s'effectuant au sein des mélanosomes. La synthèse de la mélanine (mélanogénèse) est augmentée sous l'action des UV. La fonction physiologique du bronzage qui s'ensuit est donc de protéger la peau contre les UV.

Divers dysfonctionnements du mécanisme de production de la mélanine (dus à un excès d'agressions extérieures, aux perturbations hormonales ou au vieillissement) provoquent l'apparition de taches brunes, notamment sous formes d'éphélides (taches de rousseur), de taches solaires ou de taches de sénescence.

Modifier la pigmentation naturelle de la peau est un souhait largement partagé par les femmes européennes, asiatiques, américaines, quoique à des titres divers : blancheur du teint, car trait de beauté pour les unes, ou atténuation des taches pigmentaires de sénescence révélatrices de l'âge pour les autres. En Asie comme en Europe et Amérique, la maîtrise de la pigmentation est donc un sujet sensible et l'objet d'une forte demande. Trois enzymes clés sont impliquées dans la mélanogénèse : la tyrosinase et les tyrosine-related proteins (TRP-1 et TRP-2), toutes trois sont des glycoprotéines localisées dans la membrane des mélanosomes. Sur les trois, la tyrosinase est l'enzyme limitante en catalysant les deux premières étapes de la formation du pigment : ortho-hydroxylation de la tyrosine en L-DOPA puis oxydation de cette dernière en dopaquinone. TRP-1 et TRP-2 interviendraient en partie par une stabilisation de la tyrosine hydroxylase.

D'autre part, il est connu que la stimulation de la mélanogénèse passe par une augmentation des taux intracellulaires en AMPc, qui régulent à leur tour une activité protéine kinase PKC-b dont la capacité à phosphoryler la tyrosinase va être déterminante pour la synthèse mélanique. A l'appui de ce mécanisme on observera que l'irradiation UV augmente très significativement la PKC-b sur les mélanocytes en culture.

Enfin, le rôle joué par le calcium intracellulaire dans le métabolisme du mélanocyte est sans doute aussi à prendre en compte.

Pour agir sur la pigmentation de la peau, il est donc possible d'envisager de dégrader la mélanine, de proposer des inhibiteurs de la mélanogénèse qui interagissent sur les diverses cibles décrites plus haut, voire d'inhiber la distribution de mélanine dans les couches de cellules épidermiques. Cependant, la cible la plus couramment choisie est certainement la tyrosine hydroxylase puisqu'elle constitue une étape limitante du processus.

Longtemps, la dépigmentation ou l'éclaircissement de la peau ont été traités par des produits très puissants tels que l'hydroquinone, les composés phénoliques soufrés ou non, l'acide ascorbique, mais non dénués d'effets irréversibles d'hypopigmentation et d'irritation. Tous ces produits sont à utiliser dans un contexte efficacité / tolérance qui n'est pas adapté à la cosmétique.

En cosmétique, le problème a été abordé par l'utilisation des dérivés rétinoïdes, des AHA, de l'acide kojique, de l'arbutine.

L'hydroquinone, l'arbutine et l'acide kojique ont été développés pour l'inhibition compétitive de la tyrosinase ou inhibition de l'activité catalytique par chélation de l'ion cuivre indispensable à son fonctionnement. Mais leur utilisation est délicate et peut entraîner des effets secondaires.

Le document US 4 258 058 décrit l'utilisation de tyramine ou de dérivés en tant que médicament administré par voie orale ou par injection pour inhiber l'agrégation des thrombocytes.

Des produits cosmétiques innovants, efficaces *in vivo,* et non toxiques sont donc fortement souhaités.

Nous avons découvert de façon tout à fait surprenante que certains dérivés de la tyramine possèdent un fort pouvoir d'inhibition de la mélanogénèse, supérieur à la tyramine elle-même, décrite dans la demande de brevet FR-A-2 813 188.

L'invention faisant l'objet de cette demande réside dans le fait que nous avons découvert et démontré que des composés dérivés de la tyramine de réduisent la production de mélanine de façon efficace et non toxique. Les dérivés de la tyramine objets de la présente demande présentent également un intérêt pour leur meilleure biodisponibilité, solubilité, activité, stabilité, ou meilleur profil toxicologique.

La présente divulgation concerne des compositions cosmétiques ou dermopharmaceutiques comprenant un excipient cosmétiquement acceptable et, seul ou en association, un composé répondant à la formule **I** ci-dessous : 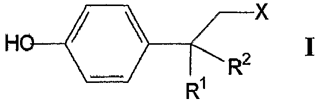 dans laquelle :
le groupe X représente un groupe -NR³R⁴ ou -N=CR⁵R⁶,
les groupes R¹ et R², qui peuvent être identiques ou différents, représentent chacun un atome d'hydrogène ou d'halogène, ou un groupe alkyle, aryle, aralkyle, acyle, alcool ou alkoxy,
les groupes R³ et R⁴, qui peuvent être identiques ou différents, représentent chacun un atome d'hydrogène, un groupe alkyle, aryle, aralkyle, acyle, sulfonyle, sucre,
les groupes R⁵ et R⁶, qui peuvent être identiques ou différents, représentent chacun un atome d'hydrogène, un groupe alkyle, aryle ou aralkyle.

Les composés de formule générale I peuvent exister sous leur forme libre, ou sous la forme d'un sel avec un acide cosmétiquement acceptable. La présente invention comprend aussi bien les formes libres ou les sels de ces composés.

La présente invention ne concerne pas les compositions cosmétiques ou dermopharmaceutiques renfermant la tyramine (formule **I**, X = -NR³R⁴, R¹ = R² = R³ = R⁴ = H) et ses dérivés greffés sur le OH ou sur le NH₂ par toute chaîne alkoyle, linéaire ou branchée, saturée ou insaturée, hydroxylée ou soufrée ou non, contenant de 1 à 24 atomes de carbone ni celles renfermant la synéphrine (formule **I**, X = -NR³R⁴, R¹ = OH, R² = R³ =H, R⁴ = CH₃).

Par le terme « acide cosmétiquement acceptable » on entend au sens de la présente invention tout acide non toxique, y compris les acides organiques et inorganiques. De tels acides incluent les acides acétique, p-amino benzoïque, ascorbique, aspartique, benzènesulfonique, benzoïque, bis-méthylènesalicylique, bromhydrique, chlorhydrique, cinnamique, citraconique, citrique, éthanedisulfonique, fumarique, gluconique, glutamique, glyconique, itaconique, lactique, maléique, malique, mandélique, méthanesulfonique, mucique, nitrique, oxalique, palmitique, pamoïque, pantothénique, phosphorique, propionique, salicylique, stéarique, succinique, sulfamique, sulfurique, tartarique et paratoluènesulfonique. Les acides chlorhydrique et acétique sont particulièrement préférés.

Par le terme « groupe alkyle », on entend au sens de la présente invention tout groupe alkyle de 1 à 20 atomes de carbone linéaire ou ramifié, substitué ou non (notamment par un alcool, un acide carboxylique, une amine), saturé ou insaturé. En particulier un groupe alkyle est le groupe méthyle.

Par le terme « groupe aryle », on entend au sens de la présente invention un ou plusieurs cycles aromatiques ayant chacun de 5 à 8 atomes de carbone, pouvant être accolés ou fusionnés, substitués ou non. En particulier, les groupes aryles peuvent être des groupes phényle, ou naphtyle et les substituants des atomes d'halogène, des groupes alkoxy tels que définis ci-dessus, des groupes alkyles tels que définis ci-dessus ou le groupe nitro.

Par le terme « groupe aralkyle », on entend au sens de la présente invention tout groupe aryle tel que défini ci-dessus, lié par l'intermédiaire d'un groupe alkyle tel que défini ci-dessus. En particulier un groupe aralkyle est le groupe benzyle.

Par le terme « groupe alkoxy », on entend au sens de la présente invention tout groupe -OR⁷ où R⁷ peut être un groupe alkyle, aryle, aralkyle, acyle, sulfonyle, sucre, tels que définis ci-dessus.

Par le terme « groupe acyle », on entend au sens de la présente invention tout groupe -C=OR⁸ où R⁸ peut être un groupe alkyle, aryle, aralkyle, amine, tels que définis ci-dessus. En particulier, un groupe acyle est le groupe acétyle (R⁸ = -CH₃).

Par le terme « groupe amine », on entend au sens de la présente invention tout groupe -NR⁹R¹⁰ où R⁹ et R¹⁰, qui peuvent être identiques ou différents, représentent chacun un atome d'hydrogène un groupe alkyle, aryle, aralkyle, acyle, sulfonyle, sucre, tels que définis ci-dessus.

Par le terme « groupe sulfonyle », on entend au sens de la présente invention tout groupe -SO₂R¹¹ où R¹¹ peut être un groupe alkyle, aryle, aralkyle, alkoxy, amine, tels que définis ci-dessus. En particulier, des groupe sulfonyles sont les groupes mésyle (R¹¹ = -CH₃), triflyle (R¹¹ = - CF₃), ou tosyle (R¹¹ = -Ph-CH₃).

Par le terme « groupe sucre » on entend au sens de la présente invention tout groupe hexose, oses et osides. En particulier, des groupes sucres sont les groupes glucose, arabinose, fructose, galactose, mannose, maltose, lactose, saccharose, cellobiose.

Lesdits composés de formule **I** peuvent posséder un centre d'asymétrie et peuvent donc exister sous forme d'isomères optiques. La présente invention comprend aussi bien ces isomères soit séparément soit en tant que mélange.

Des compositions cosmétiques ou dermopharmaceutiques particulièrement avantageuses selon l'invention sont celles renfermant l'octopamine (formule **I**, X = -NR³R⁴, R¹ = OH, R² = R³ = R⁴ = H), la N-succinyl-tyramine (formule **II**), la N-tosyl-tyramine (formule **III**) ou la N,N'-bis-tyramine-urée (formule **IV**).

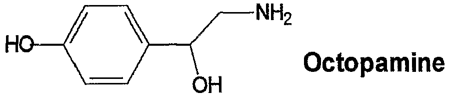

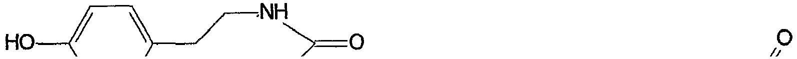

La présente divulagation concerne également le mode de préparation de ces composés de formule générale **I**. La préparation de ces molécules consiste notamment à faire réagir la tyramine ou l'octopamine ou un de leurs sels, en présence ou non de base, avec un dérivé carbonylé ou sulfonylé, en présence ou non d'un réactif de couplage utilisé en synthèse peptidique (notamment carbodiimide, acylimidazole, chloroformate, BOP, CDI, DCC, EEDQ, HTBU, PyBOP®, PyBroP®, TBTU, WSC.HCl, disponibles par exemple chez Novabiochem). Ce dérivé carbonylé ou sulfonylé peut être en particulier un aldéhyde, un ester activé ou non, un chlorure d'acide carboxylique ou sulfonique, un anhydride, un isocyanate.

La tyramine et l'octopamine, molécules de départ des synthèses, sont disponibles dans le commerce ou peuvent être préparés à partir de produits de départ disponibles dans le commerce en utilisant des procédés connus.

Les composés de formule **I**, **II**, **III** ou **IV** préférés sont ceux obtenus par le procédé décrit ci-dessus, à partir d'un sel de tyramine.

Les composés de formule I, II, III ou IV sont employés dans les compositions cosmétiques et dermopharmaceutiques conformes à l'invention à des concentrations qui peuvent varier entre 0,0001% (p/p) et 50% (p/p), préferentiellement entre 0,001% (p/p) et 20% (p/p).

Lesdits composés peuvent être utilisés individuellement ou en pré-mélange dans toute forme galénique telle que : des lotions, des laits ou des crèmes émollientes, des laits ou des crèmes pour les soins de la peau ou des cheveux, des crèmes, des lotions ou des laits démaquillants, des bases de fond de teint, des lotions, des laits ou des crèmes anti-solaires, des lotions, des laits ou des crèmes de bronzage artificiel, des crèmes ou des mousses de rasage, des lotions après-rasage, des shampoings, des rouges à lèvres, des mascaras ou des vernis à ongles.

Les compositions selon la présente divulgation peuvent également se présenter sous la forme de bâtons pour les lèvres destinés soit à les colorer, soit à éviter les gerçures, ou de produits de maquillage pour les yeux ou des fards et fonds de teint pour le visage.

Lorsque les compositions selon la présente divulgation se présentent sous la forme d'émulsions de type eau-dans-l'huile ou huile-dans-l'eau, la phase grasse est essentiellement constituée d'un mélange de corps gras d'extraction ou de synthèse, avec au moins une huile, et éventuellement un autre corps gras. La phase grasse des émulsions peut constituer de 5 à 60% du poids total de l'émulsion.

La phase aqueuse desdites émulsions constitue de préférence de 30 à 85% du poids total de l'émulsion. La proportion de l'agent émulsionnant peut être comprise entre 1 et 20%, et de préférence entre 2 et 12% du poids total de l'émulsion. Lorsque les compositions selon l'invention se présentent sous forme de lotions huileuses, oléoalcooliques ou hydroalcooliques, elles peuvent constituer, par exemple, des lotions antisolaires contenant un filtre absorbant les rayons UV, des lotions adoucissantes pour la peau ; les lotions huileuses peuvent en outre constituer des huiles moussantes contenant un tensioactif oléosoluble, des huiles pour le bain, etc.

Parmi les principaux adjuvants pouvant être présents dans les compositions selon l'invention, on peut citer les solvants organiques ou hydroglycoliques, y compris le MP-diol et les polyglycérines, les corps gras d'extraction ou de synthèse, les épaississants ioniques ou non ioniques, les adoucissants, opacifiants, stabilisants, émollients, les silicones, α- ou β-hydroxyacides, les agents anti-mousse, les agents hydratants, les vitamines, parfums, conservateurs, séquestrants, colorants, les polymères gélifiants et viscosants, les tensioactifs et émulsifiants, les autres principes actifs hydro- ou liposolubles, les extraits végétaux, extraits tissulaires, extraits marins, filtres solaires, les antioxydants.

Les mono-ou poly-alcools plus particulièrement préférés sont choisis parmi l'éthanol, l'isopropanol, le propylène-glycol, le glycérol et le sorbitol.

A titre de corps gras, parmi les huiles minérales, on peut citer l'huile de vaseline ; parmi les huiles animales, les huiles de baleine, de requin, de phoque, de menhaden, de foie de flétan, de morue, de thon, de tortue, de pied de boeuf, de pied de cheval, de pied de mouton, de vison, de loutre, de marmotte, etc. ; parmi les huiles végétales, les huiles d'amande, de germes de blé, de jojoba, de sésame, de tournesol, de palme, de noix, de karité, de shoréa, de macadamia, de pépins de cassis et similaires.

Parmi les esters d'acides gras, on peut utiliser des esters d'acide en C₁₂ à C₂₂ saturés ou insaturés et d'alcools inférieurs comme l'isopropanol ou le glycérol ou d'alcools gras en C₈ à C₂₂, linéaires ou ramifiés, saturés ou insaturés ou encore d'alcanediols-1,2 en C₁₀-C₂₂.

On peut également citer comme corps gras, la vaseline, la paraffine, la lanoline, la lanoline hydrogénée, le suif, la lanoline acétylée, les huiles de silicone.

Parmi les cires, on peut citer la cire de Sipol, la cire de lanoline, la cire d'abeille, la cire de Candelila, la cire monocristalline, la cire de Carnauba, le spermaceti, le beurre de cacao, le beurre de karité, les cires de silicone, les huiles hydrogénées concrètes à 25°C, les sucroglycérides, les oléates, myristates, linoléates et stéarates de calcium, magnésium et aluminium. Parmi les alcools gras, on peut citer les alcools laurique, cétylique, myristique, stéarique, palmitique, oléique, et les alcools de GUERBET comme le 2-décyltétradécanol ou le 2-hexyldécanol. A titre d'émulsionnants, parmi les alcools gras polyoxyéthylénés, on peut citer les alcools laurique, cétylique, stéarylique et oléique comportant de 2 à 20 moles d'oxyde d'éthylène et parmi les alcoyléthers de glycérol, les alcools en C₁₂-C₁₈ comportant de 2 à 10 moles de glycérol. Il peut être aussi utile d'utiliser des épaississants tels que les dérivés de cellulose, les dérivés de d'acide polyacrylique, les gommes de guar ou de caroube ou la gomme de xanthane.

Les compositions selon la présente divulgation peuvent également contenir des adjuvants habituellement utilisés en cosmétique ou en dermatologie et notamment des produits hydratants, des adoucissants, des produits pour le traitement d'affections cutanées, des filtres solaires, des germicides, des colorants, des conservateurs, des parfums et des propulseurs.

Lorsque les compositions selon la présente divulgation sont des dispersions, il peut s'agir de dispersions de lécithine dans l'eau en présence de tensioactif ou encore de dispersions aqueuses de sphérules lipidiques, constituées de couches moléculaires organisées enfermant une phase aqueuse encapsulée. On peut citer, à cet effet, comme composés lipidiques, les alcools et diols à longue chaîne, les stérols tels que le cholestérol, les phospholipides, les cholestéryl sulfate et phosphate, les amines à longue chaîne et leurs dérivés d'ammonium quaternaire, les dihydroxyalkylamines, les amines grasses polyoxyéthylénées, les esters d'aminoalcools à longue chaîne, leurs sels et dérivés d'ammonium quaternaire, les esters phosphoriques d'alcools gras tels que le dicétylphosphate acide ou son sel de sodium, les alkylsulfates tels que le cétylsulfate de sodium, les acides gras sous forme de sels ou encore les lipides du type de ceux décrits dans les brevets français n° 2 315 991, 1 477 048 et 2 091 516 ou dans les demandes de brevet international WO 83/01 571 et WO 92/08 685.

On peut par exemple utiliser comme autres lipides, des lipides comportant une chaîne lipophile longue contenant 12 à 30 atomes de carbone, saturée ou insaturée, ramifiée ou linéaire, par exemple une chaîne oléique, lanolique, tétradécylique, hexadécylique, isostéarylique, laurique ou alcoylphénylique. Le groupement hydrophile de ces lipides peut être un groupement ionique ou non-ionique. A titre de groupements non-ioniques, on peut citer des groupements dérivés de polyéthylèneglycol. On peut aussi utiliser avantageusement comme lipides formant la phase lamellaire, des éthers de polyglycérol tel que ceux décrits dans les brevets français n°1 477 048, 2 091 516, 2 465 780 et 2 482 128.

A titre de groupement ionique, on peut avantageusement utiliser un groupement dérivé d'un composé amphotère, anionique ou cationique. D'autres lipides décrits dans la demande de brevet international WO 83/01 571 comme pouvant être utilisés pour la formation de vésicules sont les glycolipides comme le lactosylcéramide, le galactocérébroside, les gangliosides et le trihexosylcéramide, ainsi que les phospholipides tels que le phosphatidylglycérol et le phosphatidylinositol.

Les substances actives peuvent être des substances ayant un intérêt pharmaceutique, alimentaire ou ayant une activité cosmétique. Lorsqu'elles sont hydrosolubles, elles peuvent être dissoutes de façon homogène ou elles sont dans la phase aqueuse encapsulée à l'intérieur des vésicules. Les substances hydrosolubles ayant une activité cosmétique et/ou pharmaceutique peuvent être des produits destinés aux soins ou aux traitements de la peau et du cheveu tels que par exemple des humectants comme la glycérine, le sorbitol, le pentaérythritol, l'acide pyrrolidone carboxylique et ses sels ; des agents de brunissage artificiel tels que la dihydroxyacétone, l'érythrulose, le glycéraldéhyde, les γ-dialdéhydes tels que l'aldéhyde tartique, ces composés étant éventuellement associés à des colorants ; des filtres solaires hydrosolubles ; des antiperspirants, des déodorants, des astringents, des produits rafraîchissants, toniques, cicatrisants, kératolytiques, dépilatoires, des eaux parfumées ; des extraits de tissus végétaux, tels que les polysaccharides ; des colorants hydrosolubles ; des agents antipelliculaires ; des agents antiséborrhéiques, des oxydants tels que des agents de décoloration comme l'eau oxygénée ; des réducteurs tels que l'acide thioglycolique et ses sels.

On peut citer également les vitamines, les hormones, les enzymes, telles que la superoxyde dismutase, les vaccins, les anti-inflammatoires tels que l'hydrocortisone, les antibiotiques, les bactéricides, les agents cytotoxiques ou anti-tumoraux.

Lorsque les substances actives sont liposolubles, elles peuvent se trouver incorporées dans les feuillets des vésicules. Elles peuvent être choisies dans le groupe formé par les filtres solaires liposolubles, les substances destinées à améliorer l'état des peaux sèches ou séniles, les tocophérols, les vitamines E, F ou A et leurs esters, l'acide rétinoïque, les antioxydants, les acides gras essentiels, l'acide glycyrrhétinique, les kératolytiques et les caroténoïdes.

Les composés de formule I, II, III et IV peuvent être utilisés dans les compositions cosmétiques conformes à l'invention soit en ajout individuel, soit en tant que pré-mélange dans un excipient convenable, et utilisés sous forme de solution, de dispersion, d'émulsion, de pâte ou de poudre. Ils peuvent individuellement ou ensemble être véhiculés par des vecteurs cosmétiques tels que les macro-, micro- ou nanocapsules, des liposomes ou des chylomicrons, les macro-, micro- ou nanoparticules ou microéponges. Ils peuvent être également adsorbés sur des polymères organiques poudreux, les talcs, bentonites et autres supports minéraux.

Ils peuvent être utilisés dans une forme quelconque, ou sous une forme liée ou incorporée ou absorbée ou adsorbée à de macro-, micro- et nanoparticules, de macro-, micro- et nanocapsules, pour le traitement des textiles, fibres synthétiques ou naturelles, laines et tous matériaux susceptibles d'être utilisés pour réaliser des vêtements et sous-vêtements de jour ou de nuit destinés à être en contact avec la peau tel que les collants, sous-vêtements, mouchoirs, lingettes, pour exercer leur effet cosmétique via ce contact textile/peau et permettre une délivrance topique continue.

La présente divulgation concerne également l'utilisation des compositions cosmétiques ou dermopharmaceutiques renfermant les composés de formule générale **I**, à l'exception de la tyramine et de ses dérivés greffés sur le OH ou sur le NH₂ par toute chaîne alkoyle, linéaire ou branchée, saturée ou insaturée, hydroxylée ou soufrée ou non, contenant 1 à 24 atomes de carbone, et de la synéphrine (formule **I**, X = -NR³R⁴, R¹ = OH, R² = R³ =H, R⁴ = CH₃), pour la réduction de la production de la mélamine dans l'objectif d'une diminution de la pigmentation, en particulier pour l'éclaircissement du teint, l'atténuation des taches cutanées de sénescence, l'homogénéisation de la coloration de la peau, l'éclaircissement de toute pigmentation associée à la mélanine, y compris celle des cheveux.

La présente invention concerne aussi l'utilisation des composés de formule **I, II, III** ou **IV** et de l'octopamine, seuls ou incorporés dans des compositions cosmétiques ou dermopharmaceutiques selon l'invention, pour la préparation de médicaments pour l'inhibition de la mélanogénèse ou destinés aux soins de la peau, particulièrement à son blanchissement et à sa moindre coloration lors d'expositions aux UV naturels ou artificiels.

A titre d'illustration, suivent des exemples, non limitatifs, de la mise en oeuvre de la présente invention.

### Exemple n°1 : Synthèse de la N-succinyl-tyramine (composé II)

A une solution de chlorhydrate de tyramine (1,00 g ; 5,76 mmoles) dans 20 ml de THF sont additionnés, à température ambiante, 1 équivalent de carbonate de potassium (K₂CO₃) (0,80 g ; 5,76 mmoles) et 1,04 équivalent d'anhydride succinique (0,60 g ; 5,99 mmoles). Après une nuit d'agitation à température ambiante, le mélange est hydrolysé par addition d'eau (10 ml) et lavé par ajout de 4 g de résine amberlite IR120 (R-SO₃H) et agitation 15 minutes (pH = 0-1). Après filtration et rinçage à l'eau, le THF est évaporé à froid. 0,92 g (3,88 mmoles ; 67,3 %) de N-succinyl-tyramine sont isolés sous la forme de cristaux blancs.

C₁₂H₁₅NO₄

MM = 237,257 gmol⁻¹
Point de Fusion : 135-136°C
CHN : Calculé : 60,75 %C ; 6,35 %H ; 5,90 %N
   Trouvé : 61,33 %C ; 6,05 %H ; 5,86 %N
Infra Rouge : 3313 ; 3055 ; 2930 ; 1694 ; 1643 ; 1542 ; 1517 ; 1426 ; 1238 ; 1208 cm⁻¹

### Exemple n°2 : Synthèse de la N-tosyl-tyramine (composé III)

A une solution de chlorhydrate de tyramine (0,20 g ; 1,15 mmoles) dans 4 ml de THF sont additionnés, à température ambiante, 1 équivalent de carbonate de potassium (K₂CO₃) (0,16 g ; 1,16 mmoles) et 1,10 équivalent de chlorure de tosyle (0,12 g ; 1,27 mmoles). Après une nuit d'agitation à température ambiante, le mélange est hydrolysé par addition d'eau (4 ml) et lavé par ajout de 4 g de résine amberlite IR120 (R-SO₃H) et agitation 1 heure. Après filtration et rinçage à l'eau puis au THF, le solvant est évaporé et le solide filtré sur fritté. 0,19 g (0,65 mmoles ; 56,0 %) de N-tosyl-tyramine sont isolés sous la forme de cristaux blancs.

C₁₅H₁₇NSO₃

MM = 291,37 gmol⁻¹
Point de Fusion : 169-172°C
CHN : Calculé : 61,83 %C ; 5,88 %H ; 4,81 %N
   Trouvé : 61,94 %C ; 5,83 %H ; 4,78 %N
Infra Rouge : 3335 ; 3220 ; 1613 ; 1598 ; 1515 ; 1435 ; 1312 ; 1229 ; 1149 ; 1063 ; 913 ; 833 ; 812 cm⁻¹

### Exemple n°3 : Synthèse de la N,N'-bis-tyramine-urée (composé IV)

A une solution de chlorhydrate de tyramine (0,20 g ; 1,15 mmoles) dans 4 ml de THF sont additionnés, à température ambiante, 1 équivalent de carbonate de potassium (K₂CO₃) (0,16 g ; 1,16 mmoles) et 0,54 équivalent de carbonyl-diimidazole (0,10 g ; 0,62 mmole). Après deux jours d'agitation à température ambiante, le mélange est hydrolysé par addition d'eau (4 ml) et de THF (4 ml) et lavé par ajout de 4 g de résine amberlite IR120 (R-SO₃H) et agitation 15 minutes. Après filtration et rinçage avec 4 ml de THF puis 4 ml d'eau, le solvant est évaporé et le solide filtré sur fritté. 0,08 g (0,27 mmoles ; 43,2 %) de N,N'-bis-tyramine-urée sont isolés sous la forme de cristaux blancs.

C₁₇H₂₀N₂O₃

MM = 300,3605 gmol⁻¹
Point de Fusion : 95-98°C
Spectrométrie de masse : (m/z) = 301,2 [M+H]⁺
CHN : Calculé : 67,98 %C ; 6,71 %H ; 9,33 %N
   Trouvé : 67,66 %C ; 6,73 %H ; 9,36 %N
Infra Rouge : 3336 ; 3104 ; 2930 ; 1605 ; 1515 ; 1445 ; 1240 ; 1169 ; 1050 ; 822 cm⁻¹

| **Exemple n°4 :** Crème de jour | g/100 g |
|---|---|
| Volpo S20 | 2,4 |
| Volpo S2 | 2,6 |
| Prostéaryl 15 | 8,0 |
| Cire d'abeille | 0,5 |
| Stéaroxy diméthicone | 3,0 |
| Propylène glycol | 3,0 |
| Carbomer | 0,25 |
| Soude 30% | 0,25 |
| Octopamine | 0,1 |
| Eau & conservateurs | qsp 100g |

Cette émulsion est utilisée pour éclaircir et hydrater la peau du visage.

**Exemple n°5 : Lait corporel hydratant et éclaircissant**

| | |
|---|---|
| Crillet 3 | 2,5 |
| Novol | 0,9 |
| Fluilan | 2,5 |
| Carbopol 940 | 0,3 |
| Cire d'abeille | 2,0 |
| Soude 30% | 0,1 |
| Glycérine | 5,0 |
| N-tosyl-tyramine (**III**) | 0,01 |
| Eau & conservateurs | qsp 100g |

| **Exemple n°6 :** Gel éclaircissant | g/100g |
|---|---|
| Carbomer | 0,3 |
| Propylène glycol | 2,0 |
| Glycérine | 1,0 |
| Vaseline blanche | 1,5 |
| Cylomethicone | 6,0 |
| Crodacol C90 | 0,5 |
| Lubrajel MS | 10,0 |
| Triéthanolamine | 0,3 |
| N-succinyl-tyramine (**II**) | 0,02 |
| Eau, conservateurs, parfum | qsp 100 g |

| **Exemple n°7** : crème dépiqmentante | g/100g |
|---|---|
| Cromul EM 1207 | 2,4 |
| Volpo S2 | 2,6 |
| Prostearyl 15 | 8,0 |
| Cire d'abeille | 0,5 |
| Stéaroxy diméthicone | 3,0 |
| Propylène glycol | 3,0 |
| Carbopol 941 | 0,25 |
| Triéthanolamine | 0,25 |
| N,N'-bis-tyramine-urée (**IV**) | 0,007 |
| Eau, conservateurs, parfums | qsp 100 g |

### Exemple N° 8 : Inhibition de la synthèse de mélanine (in vitro)

L'efficacité des produits sur la mélanisation, a été testée en culture de cellules en lignée établie.

Les mélanocytes de la lignée B16 ont été utilisés dans cette expérience. Cette lignée est classiquement utilisée pour tester les variations de taux de mélanine. Les cellules sont incubées en présence du produit à tester pendant 48 heures, les cellules témoins sont incubées en milieu de culture seul.

Après 48 heures, la mélanine totale (phaéomélanine et eumélanine) présente dans les cellules est obtenue après lyse des cellules et solubilisation par la soude, le dosage est colorimétrique.

Les taux de mélanine produits en présence du produit à tester à différentes concentrations sont comparés à ceux obtenus dans les cellules témoins. Les données sont normalisées par la teneur en protéine de l'échantillon.

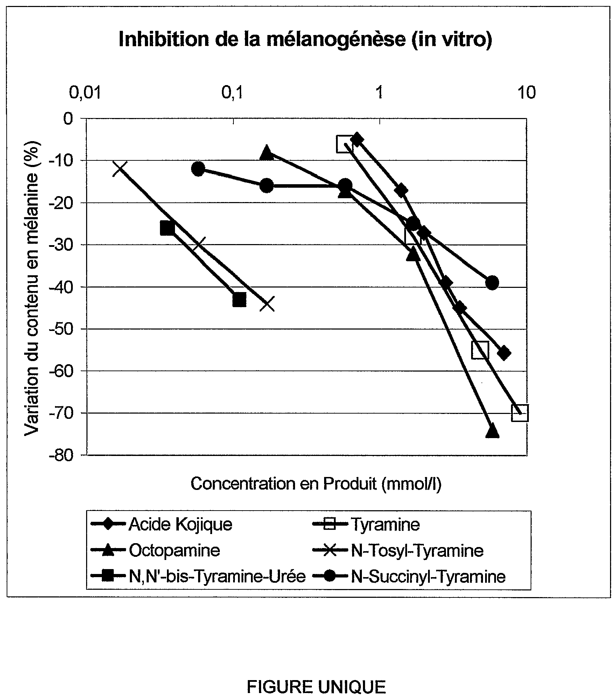

La figure unique montre la variation de synthèse de la mélanine sous l'effet d'un contact de 48 heures avec l'acide kojique (témoin positif) d'une part, et quelques dérivés de la tyramine d'autre part. L'inhibition de synthèse observée en 48 heures de contact pour ces produits est dépendante de la concentration testée. Cette inhibition varie de - 5% à - 70%. Nous montrons ainsi que tous ces composés ont des activités d'inhibition de la mélanogénèse tout à fait intéressantes, et supérieure à celle de la tyramine elle-même, notamment à faibles concentrations (≤ 0,8 mmol/l)

Les exemples de composés nouveaux dérivés de la tyramine, des compositions cosmétiques les contenant, et de leurs utilisations ne sont pas limitatifs.
Ces compositions cosmétiques ou dermopharmaceutiques peuvent également être utilisées pour la préparation de médicaments destinés aux soins de la peau, particulièrement à son blanchissement et à sa moindre coloration lors d'exposition aux UV naturels ou artificiels. En outre, les composés et compositions objets du présent brevet peuvent être utilisés pour fabriquer des tissus, textiles et vêtements à effet cosmétique, notamment pour éclaircir la peau ou les cheveux.

## Revendications

1. Utilisation d'un composé dérivé de la tyramine, seul ou en association, ou d'une composition le comprenant ainsi qu'un excipient, le composé étant choisi parmi :
- l'octopamine répondant à la formule I suivante 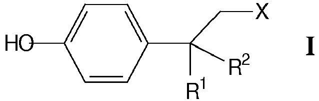 dans laquelle X = -NR³R⁴, R¹ = OH, R² = R³ = R⁴ = H,
- la N-succinyl-tyramine de formule **II** suivante : 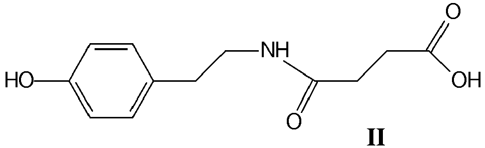
- la N-tosyl-tyramine de formule **III** suivante : 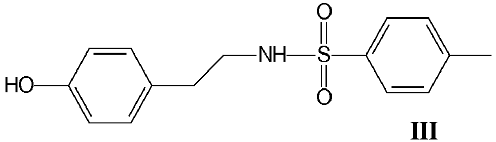
- la N, N'-bis-tyramine urée de formule **IV** suivante : 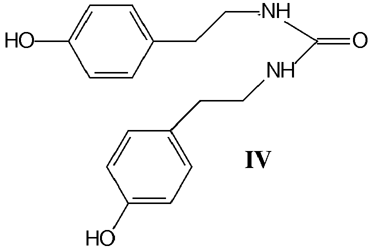
et leur sel avec un acide cosmétiquement acceptable, sous forme de leur isomère optiquement pur ou d'un mélange d'isomères,
pour un traitement cosmétique pour éclaircir le teint, atténuer les taches cutanées de sénescence, homogénéiser la coloration de la peau, éclaircir toute pigmentation associée à la mélanine, y compris celle des cheveux, ou pour une moindre coloration lors d'expositions aux UV naturels ou artificiels.

2. Utilisation selon la revendication 1, **caractérisée en ce que** ledit composé est présent à une concentration comprise entre 0,0001 % (p/p) et 50% (p/p) dans ladite composition.

3. Utilisation selon la revendication 2, **caractérisé en ce que** le composé est présent à une concentration comprise entre 0,001% (p/p) et 20% (p/p) dans ladite composition.

4. Utilisation selon l'une quelconque des revendications précédentes, **caractérisée en ce que** ledit composé est sous forme de solution, de dispersion, d'émulsion, de pâte ou de poudre, individuellement ou en pré-mélange ou est véhiculé individuellement ou en pré-mélange par des vecteurs comme les macro-, micro- ou nanocapsules, des liposomes ou des chylomicrons, des macro-, micro- ou nanoparticules, ou les microéponges, ou adsorbé sur des polymères organiques poudreux, les talcs, bentonites et autres supports minéraux.

5. Utilisation selon l'une quelconque des revendications précédentes, **caractérisée en ce que** le composé est utilisé individuellement ou en pré-mélange dans une forme galénique de lotions, laits ou crèmes émollients, laits ou crèmes pour les soins de la peau ou des cheveux, crèmes, lotions ou laits démaquillants, bases de fond de teint, lotions, laits ou crèmes anti-solaires, lotions, laits ou crèmes de bronzage artificiel, crèmes ou mousses de rasage, lotions après-rasage, shampoings, rouges à lèvres, mascaras ou vernis à ongles.

6. Utilisation selon l'une quelconque des revendications précédentes, **caractérisée en ce que** la composition contient au moins un autre ingrédient choisi parmi les : solvants organiques ou hydroglycoliques, corps gras d'extraction ou de synthèse, épaississants ioniques ou non ioniques, adoucissants, opacifiants, stabilisants, émollients, silicones, α-hydroxyacides, agents anti-mousse, agents hydratants, vitamines, parfums, conservateurs, séquestrants, colorants, polymères gélifiants et viscosants, tensioactifs et émulsifiants, autres principes actifs hydro- ou liposolubles, extraits végétaux, extraits tissulaires, extraits marins, filtres solaires et antioxydants.

7. Utilisation selon l'une quelconque des revendications précédentes, **caractérisé en ce que** ledit composé est utilisé sous une forme liée ou incorporée ou absorbée ou adsorbée à de macro-, micro- et nanoparticules, de macro-, micro- et nanocapsules, dans les textiles, fibres synthétiques ou naturelles, laines et tous matériaux susceptibles d'être utilisés pour réaliser des vêtements et sous-vêtements de jour ou de nuit, directement au contact de la peau ou des cheveux pour en permettre une délivrance topique continue.

## Patentansprüche

1. Verwendung einer Verbindung, abgeleitet von Tyramin, alleine oder in Kombination, oder einer Zusammensetzung, die sie umfasst, ebenso wie einen Hilfsstoff, wobei die Verbindung ausgewählt ist aus:
- Octopamin, das der folgenden Formel **I** entspricht, 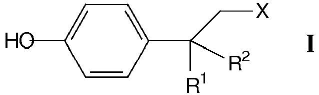 wobei X = -NR³R⁴, R¹ = OH, R² = R³ = R⁴ = H,
- N-Succinyl-Tyramin der folgenden Formel **II**: 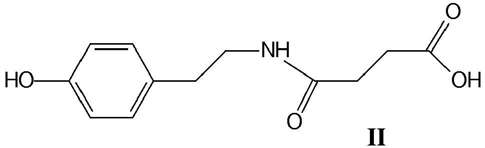
- N-Tosyl-Tyramin der folgenden Formel **III**: 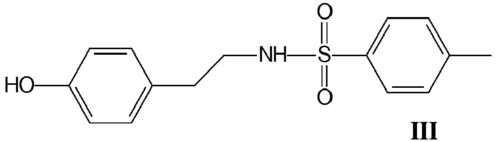
- N, N'-bis-Tyraminharnstoff der folgenden Formel **IV**: 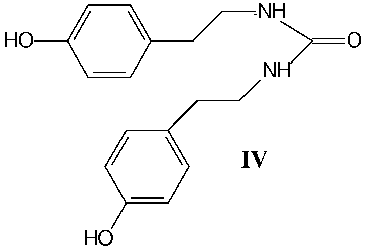
und ihrer kosmetisch annehmbaren Säure, in Form ihres optisch reinen Isomers oder einer Mischung von Isomeren,
für eine kosmetische Behandlung, um den Teint aufzuhellen, die Alterungsflecken der Haut abzuschwächen, die Färbung der Haut zu homogenisieren, jede Pigmentierung, die mit Melanin assoziiert ist, aufzuhellen, darin eingeschlossen diejenige der Haare, oder für eine geringere Färbung bei der Aussetzung an natürliche oder künstliche UV-Strahlung.

2. Verwendung nach Anspruch 1, **dadurch gekennzeichnet, dass** die Verbindung in einer Konzentration vorhanden ist, die im Bereich zwischen 0,0001 % (p/p) und 50 % (p/p) in der Zusammensetzung liegt.

3. Verwendung nach Anspruch 2, **dadurch gekennzeichnet, dass** die Verbindung in einer Konzentration vorhanden ist, die im Bereich zwischen 0,001 % (p/p) und 20 % (p/p) in der Zusammensetzung liegt.

4. Verwendung nach einem beliebigen der vorhergehenden Ansprüche, **dadurch gekennzeichnet, dass** die Verbindung die Form einer Lösung, einer Dispersion, einer Emulsion, einer Paste oder eines Pulvers, einzeln oder in Vormischung, aufweist, oder einzeln oder in Vormischung durch Vektoren wie z. B. Macro-, Micro- oder Nanokapseln, Liposome oder Chylomicrone, Macro-, Micro- oder Nanopartikel oder Microschwämme transportiert wird oder auf pulverförmigen organischen Polymeren, Talk, Bentoniten oder anderen mineralischen Trägern absorbiert wird.

5. Verwendung nach einem beliebigen der vorhergehenden Ansprüche, **dadurch gekennzeichnet, dass** die Verbindung einzeln oder in Vormischung in einer Arzneiform von Lotionen, Milch oder Weichmachercremes, Milch oder Cremes für die Pflege der Haut oder der Haare, Cremes, Lotionen oder Reinigungsmilch, Teintgrundierungen, Lotionen, Sonnenschutzmilch oder -cremes, Lotionen, Milch oder Cremes zur künstlichen Bräunung, Rasiercremes- oder -schäumen, After-Shave-Lotionen, Shampoos, Lippenstiften, Masken oder Nagellack verwendet wird.

6. Verwendung nach einem beliebigen der vorhergehenden Ansprüche, **dadurch gekennzeichnet, dass** die Zusammensetzung mindestens einen weiteren Bestandteil umfasst, ausgewählt aus: organischen oder hydro-glykolischen Lösemitteln, Extraktions- oder Synthesefettstoffen ionischen oder nicht ionischen Verdickungsmitteln, Enthärtern, Trübungsmitteln, Stabilisierungsmitteln, Weichmachern, Silikonen, α-Hydroxylsäuren, Antischaummitteln, Feuchthaltemitteln, Vitaminen, Parfüms, Konservierungsmitteln, Komplexbildnern, Farbstoffen, Gelier- oder Viskosifizierungspolymeren, Tensiden und Emulgatoren, anderen wasser- oder fettlöslichen Wirkstoffen, pflanzlichen Extrakten Gewebeextrakten, Meeresextrakten, Sonnenfiltern oder Antioxidationsmitteln.

7. Verwendung nach einem beliebigen der vorhergehenden Ansprüche, **dadurch gekennzeichnet, dass** die Verbindung in einer Form verwendet wird, die mit Macro-, Micro- und Nanopartikeln, Macro-, Micro- und Nanokapseln in Textilien, synthetischen oder natürlichen Fasern, Wolle und allen Materialien verbunden oder darin eingeschlossen oder davon absorbiert oder adsorbiert ist, die verwendet werden können, um Tages oder Nachtkleidung und Unterwäsche herzustellen, die direkt in Kontakt mit der Haut oder den Haaren ist, um eine kontinuierliche topische Verabreichung zu ermöglichen.

## Claims

1. Use of a compound derived from tyramine, alone or in combination, or a composition comprising it and an excipient, the compound being chosen from:
- octopamine corresponding to the following formula I: 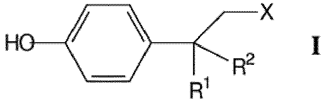 wherein X = NR³R⁴, R¹ = OH, R² = R³ = R⁴ = H,
- N-succinyl-tyramine corresponding to the following formula II: 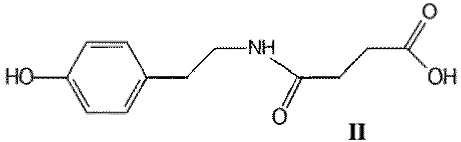
- N-tosyl-tyramine corresponding to the following formula III: 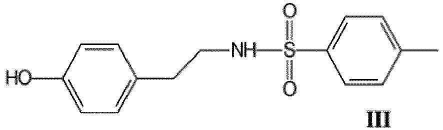
- N, N'-bis-tyramine urea corresponding to the following formula IV: 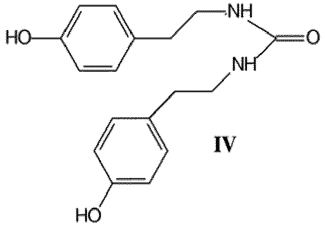
and salt thereof with a cosmetically acceptable acid, in the form of their optically pure isomer or a mixture of isomers,
for a cosmetic treatment to lighten complexion, to reduce cutaneous spots of senescence, to homogenize coloration of the skin, to lighten any pigmentation associated with melanin, including that of the hair, or for a less coloration during exposures to natural UV or artificial.

2. Use according to claim 1, **characterized in that** said compound is present at a concentration of between 0.0001% (w/w) and 50% (w/w) in said composition.

3. Use according to claim 2, **characterized in that** the compound is present at a concentration of between 0.001% (w/w) and 20% (w/w) in said composition.

4. Use according to any one of the preceding claims, **characterized in that** said compound is in the form of a solution, dispersion, emulsion, paste or powder, individually or in premix, or is conveyed individually or in premix by vectors such as macro-, micro- or nanocapsules, liposomes or chylomicrons, macro-, micro- or nanoparticles, or micro-sponges, or adsorbed on powdery organic polymers, talcs, bentonites and other mineral supports.

5. Use according to any one of the preceding claims, **characterized in that** the compound is used individually or in premix in a galenical form of lotion, milk or cream emollient, milks or creams for the care of skin or hair, cream, lotion or milk cleansing, make-up foundations, sunscreen lotions, milks or creams, artificial tanning lotions, milks or creams, shaving creams or foams, aftershave lotions, shampoos, lipsticks, mascaras or nail polish.

6. Use according to any one of the preceding claims, **characterized in that** the composition contains at least one other ingredient selected from: organic or hydroglycolic solvents, extraction or synthesis fatty substances, ionic or nonionic thickeners, softeners, opacifiers, stabilizers, emollients, silicones, α-hydroxy acids, anti-foam agents, moisturizing agents, vitamins, perfumes, preservatives, sequestering agents, colorants, gelling and viscosity polymers, surfactants and emulsifiers, other hydro- or fat-soluble active ingredients, plant extracts, tissue extracts, marine extracts, sunscreens and antioxidants.

7. Use according to any one of the preceding claims, **characterized in that** said compound is used in a form bound or incorporated or absorbed or adsorbed to macro-, micro- and nano-particles, macro-, micro- and nanocapsules, in textiles, synthetic or natural fibers, wools and any materials that may be used to make day or night clothes and underwear, directly in contact with skin or hair to enable continuous topical delivery.
